(19) Europäisches Patentamt
European Patent Office
Office européen des brevets

(11) Publication number : 0 515 141 A1

# (12) EUROPEAN PATENT APPLICATION

(21) Application number : 92304524.9

(51) Int. Cl.[5] : C07H 17/08

(22) Date of filing : 19.05.92

(30) Priority : 20.05.91 US 703063

(43) Date of publication of application : 25.11.92 Bulletin 92/48

(84) Designated Contracting States : CH DE FR GB IT LI NL

(71) Applicant : MERCK & CO. INC.
126, East Lincoln Avenue P.O. Box 2000
Rahway New Jersey 07065-0900 (US)

(72) Inventor : Wilkening, Robert R.
11 Salter Place
Maplewood, NJ 07042 (US)

(74) Representative : Thompson, John Dr.
Merck & Co., Inc. European Patent
Department Terlings Park Eastwick Road
Harlow, Essex CM20 2QR (GB)

(54) **Novel process for the preparation of 8A-aza-8A-homoerythromycin cyclic lactams.**

(57) A method of synthesizing cyclic lactams of the general structural formula

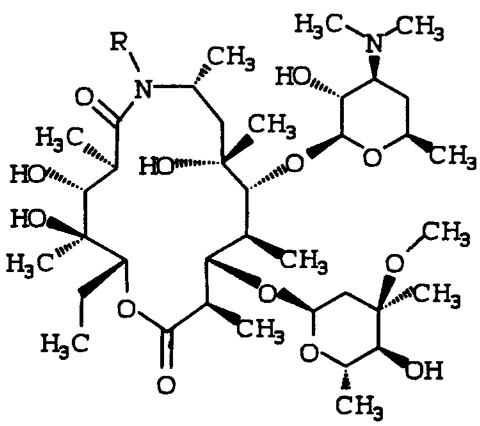

wherein R is hydrogen or $C_{1-10}$ alkyl. These compounds are macrolides useful as antibiotics and as intermediates for the synthesis of other macrolide antibiotics.

EP 0 515 141 A1

BACKGROUND OF THE INVENTION

The present invention relates to a novel method of preparing novel chemical compounds having antibacterial activity, which are useful in the therapy of bacterial infections in mammals. The compounds made by the method of this invention are also themselves useful as intermediates in the synthesis of other antibacterial compounds. More specifically, the method of preparation of the present invention, and the method of use of the novel starting material for the preparation, relates to derivatives of the well-known macrolide antibiotic, erythromycin A, the compound of the structure:

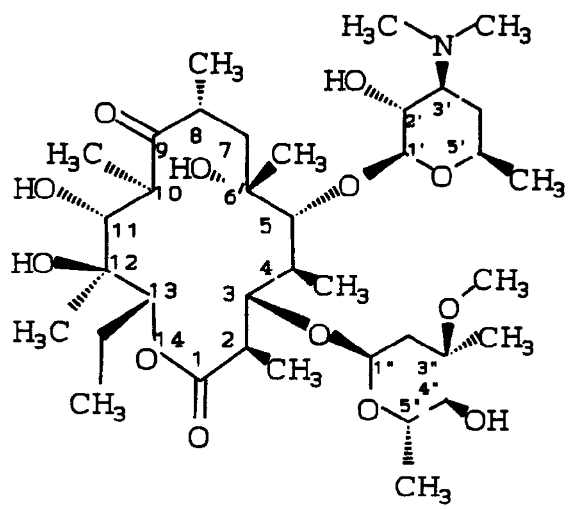

Even more specifically, the invention relates to a method of making cyclic lactams of the general structural formula:

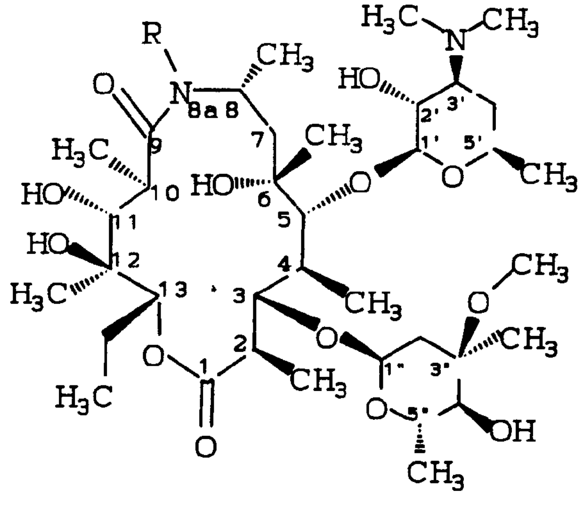

wherein R is hydrogen or $C_{1-10}$ alkyl.

SUMMARY OF THE INVENTION

The lactams obtained by the process of the invention are obtained by reacting 9-deoxo-9(Z)-hydroxyiminoerythromycin A of the structural formula

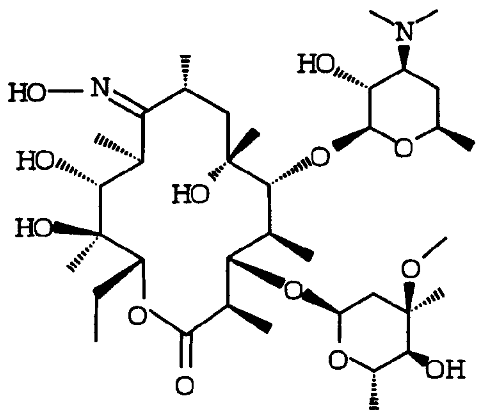

with a suitable acid, preferably a sulfonyl halide of the general formula $RSO_2X$, where R is a suitable aryl group and X is a halogen. More preferably, R is toluyl, and most preferably it is p-toluyl while X is Cl. This reaction can be controlled as to the final compound obtained by the selective addition of solvents and control of pH during the reaction. For example, the cyclic lactam and the 6, 9 cyclic iminoether are obtained by the use of acetone and aqueous sodium bicarbonate, while the 9, 12 cyclic iminoether is obtained when pyridine and ethyl ether are added.

DETAILED DESCRIPTION OF THE INVENTION

The overall process is illustrated in the following flow sheet.

## FLOWSHEET

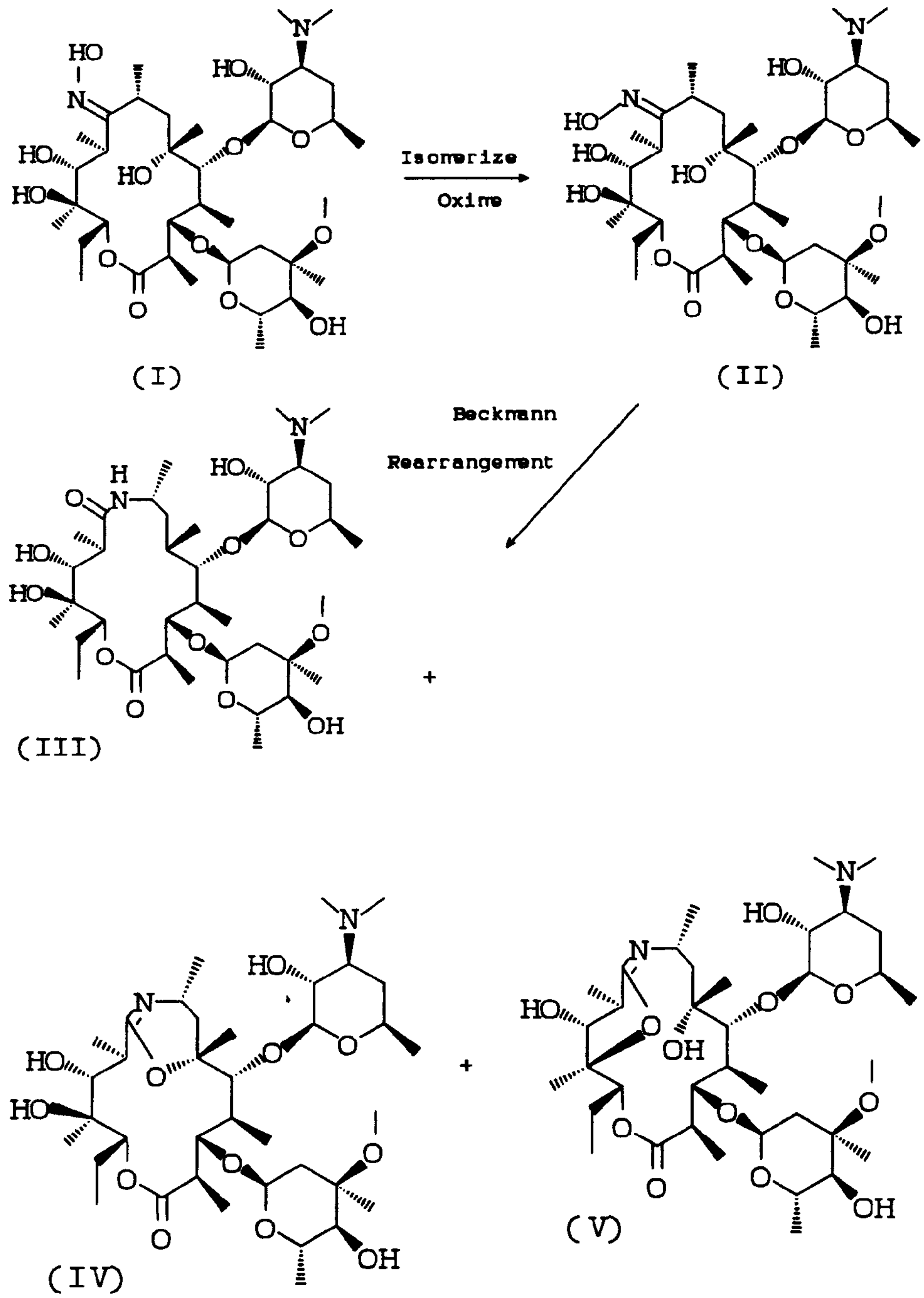


(I) (II) (III) + (IV) + (V)

(9Z)-9-Deoxo-9-hydroxyiminoerythromycin A is used as the starting compound in the synthesis of 8a-aza-8a-homoerythromycin cyclic lactams. This compound is novel and is obtained by an isomerization of (9E)-9-Deoxo-9-hydroxyiminoerythromycin A. The (E) geometric isomer of the formula:

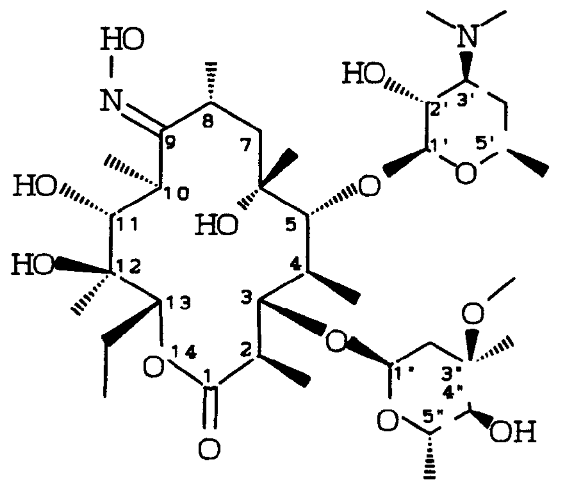

(I)

is reacted with a suitable base in the presence of a protic or an aprotic solvent. Preferably, the base is an alkali metal hydroxide, and most preferably is lithium hydroxide. The solvent is preferably an alcohol and most preferably is ethanol. Optimization of the isomerization step requires a base and solvent combination sufficient to substantially deprotonate the hydroxyimino group at position 9 of (I). Furthermore, the oxime anion must be reasonably stable under the reaction conditions for the time period required to complete the isomerization process. Upon addition of the base to (I), an equilibrium condition is created as shown in the following equation:

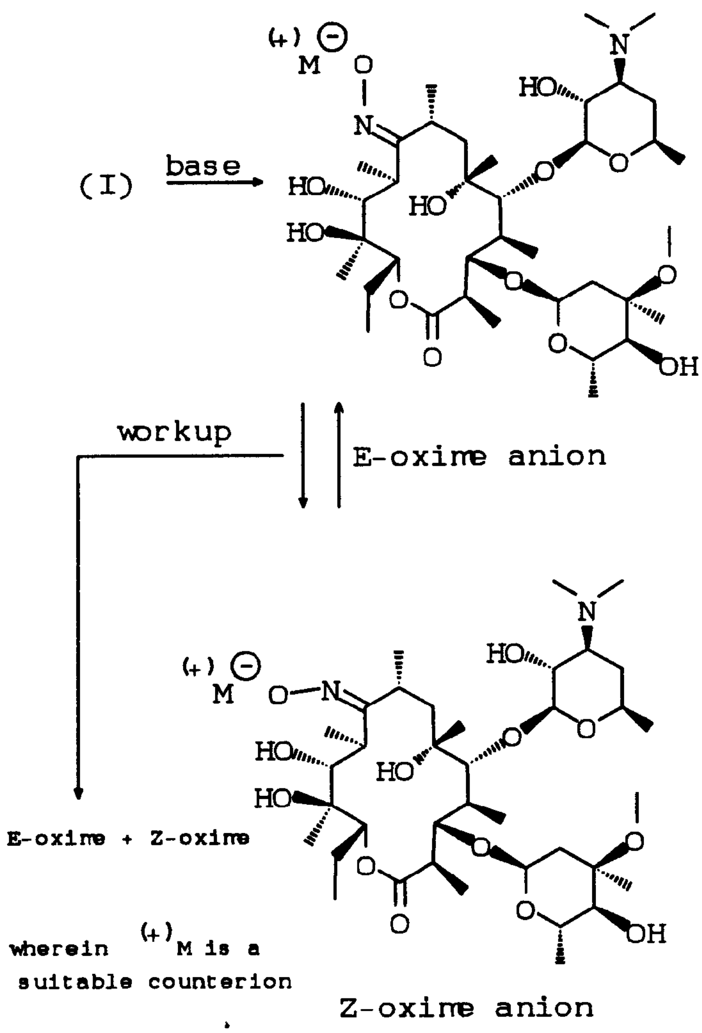

The workup performed on the anions includes protonation of the oxime anions to give the neutral oxime product mixture from which the desired Z-isomer is isolated by crystallization or by chromatography followed by crystallization.

The relative amounts of E and Z oxime anions (and neutral oximes after the workup) in the equilibrium mixture can be controlled and depends on a number of factors. These include (a) the strength and quantity of the base reagent, (b) the size and polarizability of the counterion $^+M$, (c) the reaction solvent, and (d) the reaction temperature.

Suitable bases include hydroxides, alkoxides, carbonates, metal amides, amines and metal hydrides.

The following list of reagents is given to illustrate suitable bases and solvents, although the list is not to be taken as exhaustive and other bases and solvents known to those of ordinary skill in the art are not excluded. Preferred bases and solvents are indicated by an asterisk and most preferred bases are indicated by a dagger.

Bases

1. Hydroxides

| | | |
|---|---|---|
| * † | LiOH | lithium hydroxide |
| * † | NaOH | sodium hydroxide |
| * | KOH | potassium hydroxide |
| | CsOH | cesium hydroxide |
| | $Ca(OH)_2$ | calcium hydroxide |
| | $Mg(OH)_2$ | magnesium hydroxide |
| * | $Me_4NOH$ | tetramethylammonium hydroxide |
| | $BnMe_3NOH$ | benzyltrimethylammonium hydroxide |
| | $Et_4NOH$ | tetraethylammonium hydroxide |
| | $Bu_4NOH$ | tetralbutylammonium hydroxide |

2. Alkoxides

| | | |
|---|---|---|
| * † | LiOMe | lithium methoxide |

| | | |
|---|---|---|
| * † | LiOEt | lithium ethoxide |
| | LiOiPr | lithium isopropoxide |
| | LiOnBu | lithium n-butoxide |
| | LiOsBu | lithium sec-butoxide |
| * † | NaOMe | sodium methoxide |
| * † | NaOEt | sodium ethoxide |
| | NaOPr | sodium n-propoxide |
| | NaOiPr | sodium iso-propoxide |
| | NaOnBu | sodium n-butoxide |
| | NaOsBu | sodium sec-butoxide |
| | NaOtBu | sodium tert-butoxide |
| | $NaOSiMe_3$ | sodium trimethylsilanoate |
| | KOMe | potassium methoxide |
| * | KOEt | potassium ethoxide |
| | KOtBu | potassium tert-butoxide |
| | KOSiMe3 | potassium trimethylsilanoate |
| | KOsBu | potassium sec-butoxide |
| | CsOtBu | cesium tert-butoxide |
| | $Ca(OMe)_2$ | calcium methoxide |
| * | $Mg(OEt)_2$ | magnesium ethoxide |
| | $Ti(OEt)_4$ | titanium (IV) ethoxide |
| | $Ti(OiPr)_4$ | titanium (IV) isopropoxide |
| | $BnMe_3NOMe$ | benzyltrimethylammonium-methoxide |

3. Carbonates

| | | |
|---|---|---|
| | $K_2CO_3$ | potassium carbonate |
| * | $Cs_2CO_3$ | cesium carbonate |
| | $Na_2CO_3$ | sodium carbonate |

4. Amides (for use in aprotic solvents)

| | | |
|---|---|---|
| | $LiNH_2$ | lithium amide |
| | $LiNMe_2$ | lithium dimethylamide |

| | | |
|---|---|---|
| * | $LiNiPr_1$ | lithium diisopropylamide |
| | $LiN(C_6H_{11})_2$ | lithium dicyclohexylamide |
| | $LiN(SiMe_3)_2$ | lithium bis(trimethylsilyl) amide |
| | $NaNH_2$ | sodium amide |
| | $KN(SiMe_3)_2$ | potassium bis(trimethylsilyl) amide |

5. Amines

| | | |
|---|---|---|
| * | TMG | 1,1,3,3-tetramethyl guanidine |
| | DBU | 1,8-diazabicyclo[5.4.0] undec-7-ene |
| | proton sponge | 1,8-bis(dimethylamino)-naphthalene |

6. Hydrides (for use in aprotic solvents)

| | | |
|---|---|---|
| | LiH | lithium hydride |
| * | NaH | sodium hydride |
| | KH | potassium hydride |

7. Solvents

a. Protic

$H_2O$ (generally used in combination with an alcohol solvent)

| | | |
|---|---|---|
| * † | MeOH | methanol |
| * † | EtOH | ethanol |
| * | iPrOH | isopropanol |
| | n-BuOH | normal-butanol |
| | s-BuOH | sec-butanol |
| | t-BuOH | tert-butanol |

b. Aprotic

i. Nonpolar (as a group, these are generally used in combination with a protic or polar solvent)

| | | |
|---|---|---|
| | $Et_2O$ | diethyl ether |
| | THF | tetrahydrofuran |
| | DME | dimethoxyethane |
| | PhMe | toluene |
| | $CH_2Cl_2$ | dichloromethane |
| | $CHCl_3$ | chloroform |

ii. Polar

| | | |
|---|---|---|
| * | DMF | dimethylformamide |
| | DMAC | dimethylacetamide |
| | DMI | 1,3-dimethyl-2-imidazolidinone |
| * | NEP | 1-ethyl-2-pyrrolidinone |
| * | NMP | 1-methyl-2-pyrrolidinone |
| | HMPA | hexamethylphosphoramide |
| | $MeNO_2$ | nitromethane |
| * | MeCN | acetonitrile |
| | dioxane | |
| | pyridine | |
| * | DMSO | dimethylsulfoxide |

Preferably, the isomerization to obtain the (Z) isomer is carried out at a concentration of 1-25% w/v of E-oxime to solvent, and most preferably at 10% w/v. The amount of base used is preferably 1.0-10.0 molar equivalents based on the amount of starting E-oxime, more preferably 1.0-3.0 molar equivalents, and most preferably 2.0 molar equivalents. The reaction is generally run at a temperature of from 0°C to 80°C, and more preferably at 22-25°C. The reaction can be allowed to run for 0.5 hours - 20 days, but most preferably is carried out over 20-24 hours.

Beckmann Rearrangement of (9Z)-9-Deoxo-9-hydroxyiminoerythromycin A

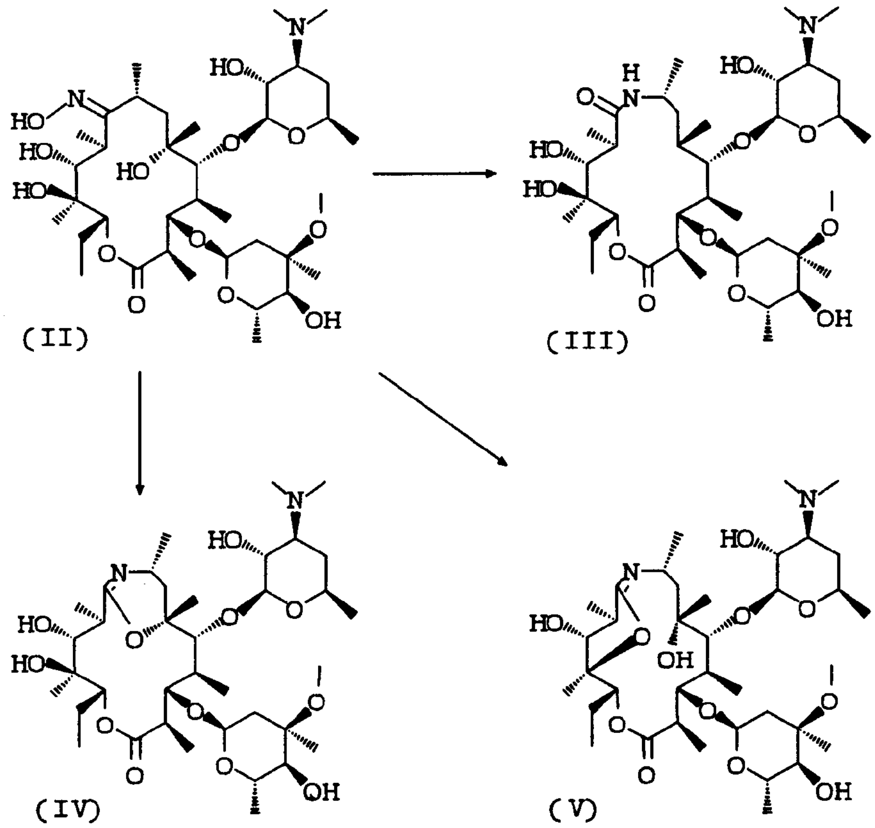

(II) (III) (IV) (V)

The conversion of (9Z)-9-deoxo-9-hydroxyiminoerythromycin A (II) to the 8a-aza-8a-homoerythromycin products (III), (IV) and (V) is accomplished by means of the Beckmann rearrangement (see "Comprehensive Organic Chemistry", I. O. Sutherland (Ed.), Pergamon Press, New York, 1979, Vol. 2, pgs. 398-400 and 967-968). In general, the Beckmann rearrangement of ketoximes leads to carboxamides and, in cyclic systems, to ring expanded lactams. The mechanism of the rearrangement involves initial conversion of the oxime hydroxyl group to a leaving group which is then lost with concomitant migration of the oxime carbon substituent that is situated anti to the leaving group. In aqueous media, the intermediate nitrilium cation thus formed usually reacts with water to afford the amide product. The nitrilium intermediate can also be trapped by other suitable nucleophiles thereby leading to imino products such as imidates and amidines.

The Beckmann rearrangement has been accomplished under a variety of acidic, neutral and basic conditions. Common acidic reagents that promote the transformation include concentrated sulfuric acid, polyphosphoric acid, thionyl chloride, phosphorous pentachloride, sulfur dioxide, and formic acid. These reagents are generally not applicable to the rearrangement of oxime (II) due to the sensitivity of the macrolide molecule, and especially the cladinose sugar residue, to acidic conditions. Efficient Beckmann rearrangement also occurs by heating the oxime with silica gel in xylene or under mildly basic conditions by heating the oxime in hexamethylphosphoramide. These conditions are not particularly valuable for the conversion of (II) to products (III), (IV) and (V) due to competing isomerization of the oxime function under the reaction conditions.

Preferred methods for effecting the Beckmann rearrangement involve initial O-acylation of the oxime group with an alkylsulfonyl halide, arylsulfonyl halide or arylsulfonic anhydride. The intermediate oxime sulfonate thus formed can be isolated or, as more commonly practiced, converted in situ to the rearranged products. The acylation and rearrangement reactions are generally performed in the presence of an organic or inorganic base. This method is particularly valuable for the conversion of oxime (II) to the rearranged products (III), (IV), and (V).

Preferred acylating reagents for effecting the rearrangement of oxime (II) include methanesulfonyl chloride, benzenesulfonyl chloride, 4-acetamidobenzenesulfonyl chloride, p-toluenesulfonyl chloride, benzenesulfonic anhydride, and p-toluenesulfonic anhydride. The reaction is carried out in the presence of an inorganic base

such as sodium bicarbonate or potassium carbonate, or in the presence of an organic base such as pyridine, 4-dimethylaminopyridine, triethylamine, or N,N-disopropylethylamine. Suitable solvents include aqueous mixtures such as aqueous acetone or aqueous dioxane and organic solvents such as dichloromethane, chloroform, ethyl acetate, diethyl ether, tetrahydrofuran, toluene, acetonitrile, and pyridine. Mixtures of organic solvents, especially those containing pyridine, are highly useful. The reaction is generally performed using 1-3 molar equivalents of the acylating agent and one or more molar equivalents of base at a raction temperature of -20°C to 50°C.

Pyridine is often used as both solvent and base.

The distribution of products resulting from Beckmann rearrangement of oxime (II) depends on the particular reaction conditions employed. For example, when the rearrangement is effected with p-toluenesulfonyl chloride and sodium bicarbonate in aqueous acetone, the major products are the lactam (III) and the 6,9-bridged iminoether (IV). When the reaction is conducted under anhydrous conditions such as p-toluenesulfonyl chloride in pyridine, the major products are the 6,9-bridged and 9,12-bridged iminoethers (IV) and (V). The ratio of products (IV) and (V) is also effected by the addition of cosolvents, by temperature, and by the initial oxime concentration.

The products of the Beckmann rearrangement of oxime (II) are conveniently purified by chromatographic methods. For example, the lactam (III) is easily separated from iminoether (IV) using column chromatograhy on silica gel or by reverse phase, high-pressure liquid chromatography. Products (IV) and (V) can also be separated by chromatographic methods, and the (V) thus obtained can be further purified by crystallization from nitromethane.

Compounds of formula (III) are basic and therefore will form acid-addition salts. Pharmaceutically acceptable acid addition salts will be non-toxic salts which can generally be prepared by methods well known to those of ordinary skill in the art.

In general, for preparation of the acid-addition salts, the compounds of formula (III) are combined with a stoichiometric amount of an appropriate acid in an inert solvent, and then the salt is recovered by solvent evaporation or by filtration if the salt precipitates spontaneously, or by precipitation using a co-solvent or a non-polar co-solvent followed by filtration.

Representative salts include the following salts:

| | |
|---|---|
| Acetate | Lactobionate |
| Benzenesulfonate | Laurate |
| Benzoate | Malate |
| Bicarbonate | Maleate |
| Bisulfate | Mandelate |
| Bitartrate | Mesylate |
| Borate | Methylbromide |
| Bromide | Methylnitrate |
| Calcium Edetate | Methylsulfate |
| Camsylate | Mucate |
| Carbonate | Napsylate |
| Chloride | Nitrate |
| Clavulanate | Oleate |
| Citrate | Oxalate |
| Dihydrochloride | Pamoate (Embonate) |
| Edetate | Palmitate |
| Edisylate | Pantothenate |
| Estolate | Phosphate/diphosphate |
| Esylate | Polygalacturonate |
| Ethylsuccinate | Salicylate |
| Fumarate | Stearate |

| | |
|---|---|
| Gluceptate | Subacetate |
| Glucoheptonate | |
| Gluconate | Succinate |
| Glutamate | Tannate |
| Glycollylarsanilate | Tartrate |
| Hexylresorcinate | Teoclate |
| Hydrabamine | Tosylate |
| Hydrobromide | Triethiodide |
| Hydrochloride | Valerate |
| Iodide | |
| Isothionate | |
| Lactate | |

As antibiotics, the compounds of formula (III) can be administered in such oral dosage forms as tablets, capsules, pills, powders, granules, elixirs, tinctures, suspensions, syrups and emulsions. Likewise, they may also be administered in intravenous, intraperitoneal, subcutaneous or intramuscular form, all using forms well known to those of ordinary skill in the pharmaceutical arts. In general, the preferred form of administration is oral. An effective but non-toxic amount of the compound can be employed as a mammalian antibiotic.

The dosage regimen utilizing the compounds of formula (III) is selected in accordance with a variety of factors including type, species, age, weight, sex and medical condition of the patient; the severity of the condition to be treated; the route of administration; the renal and hepatic function of the patient; and the particular compound or salt thereof employed. An ordinarily skilled physician or veterinarian can readily determine and prescribe the effective amount of the drug required to prevent, counter or arrest the progress of the condition.

Dosages of the compounds of formula (III), when used for the indicated effects, will range between about 0.2 mg per kg of body weight per day (mg/kg/day) to about 120 mg/kg/day and preferably 4-50 mg/kg/day. Advantageously, the compound may be administered in a single daily dose, or the total daily dosage may be administered in divided doses of two, three or four times daily.

Furthermore, the compounds of formula (III) can be administered in topical, otic or ophthalmic form via use of suitable vehicles.

Using the compounds (III), they can form the active ingredient, and are typically administered in admixture with suitable pharmaceutical diluents, excipients or carriers (collectively referred to herein as "carrier" materials)suitably selected with respect to the intended form of administration, that is, oral tablets, capsules, elixirs, syrups, and the like, and consistent with conventional pharmaceutical practices.

For instance, for oral administration in the form of a tablet or capsule, the active drug component can be combined with an oral, non-toxic, pharmaceutically acceptable, inert carrier such as lactose, starch, sucrose, glucose, methyl cellulose, magnesium stearate, dicalcium phosphate, calcium sulfate, mannitol, sorbitol, and the like; for oral administration in liquid form, the oral drug components can be combined with any oral, non-toxic, pharmaceutically acceptable inert carrier such as ethanol, glycerol, water, and the like. Moreover, when desired or necessary, suitable binders, lubricants, disintegrating agents and coloring agents can also be incorporated into the mixture. Suitable binders include starch, gelatin, natural sugars such as glucose or beta-lactose, corn sweeteners, natural and synthetic gums such as acacia, tragacanth or sodium alginate, carboxymethylcellulose, polyethylene glycol, waxes, and the like. Disintegrators include, without limitation, starch, methyl cellulose, agar, bentonite, xanthan gum, and the like.

The compounds of formula (III) can also be administered in the form of liposome delivery systems, such as small unilamellar vesicles, large unilamellar vesicles and multilamellar vesicles. Liposomes can be formed from a variety of phospholipids, such as cholesterol, stearylamine or phosphatidylcholines.

The compounds of formula (III) may also be coupled with soluble polymers as targetable drug carriers. Such polymers can include polyvinylpyrrolidone, pyran copolymer, polyhydroxypropylmethacrylamide phenyl, polyhydroxyethylaspartamide-phenol, or polyethyleneoxide-polylysine substituted with palmitoyl residues. Furthermore, the compounds of formula (III) may be coupled to a class of biodegradable polymers useful in achieving controlled release of a drug, for example, polylactic acid, polyglycolic acid, copolymers of polylactic and polyglycolic acid, polyepsilon caprolactone, polyhydroxy butyric acid, polyorthoesters, polyacetals, polydihydropyrans, polycyanoacrylates and cross-linked or amphipathic block copolymers of hydrogels.

The following examples further illustrate details for the practice of the invention. Those skilled in the art will readily understand that known variations, when taken with the alternative reagents, bases and solvents taught above, can be used in the practice of the invention.

## Example 1

### Preparation of (9E)-Deoxo-9-hydroxyiminoerythromycin A

Hydroxylamine hydrochloride (224 g, 3.23 mol) was added to a solution of erythromycin A (100 g, ca. 95% pure, 0.129 mol, obtained from Aldrich Chemical Company, Inc., Milwaukee, Wisconsin) in pyridine (500 mL). The resulting mixture was stirred at room temperature for 27 hrs., and then concentrated under vacuum at ca. 40°C. The semi-solid residue was kept under high vacuum overnight, then stirred with ethanol (600 mL) for 15 minutes and filtered. The collected solids were washed with hot (50°C) ethanol. The combined filtrate and washing was evaporated under vacuum to a pale blue foam. The foam was shaken with water (850 mL) to give a thick emulsion which was stirred rapidly at room temperature for 2.5 hours to give a filterable precipitate. The precipitate was collected, washed with water (150 mL), and dried under vacuum to give a white solid (117.7 g).

The crude oxime hydrochloride was suspended in 5% aqueous sodium bicarbonate (1000 mL) and methylene chloride (1000 mL), and the mixture was stirred while the pH was adjusted to 9.5 by addition of 5N aqueous sodium hydroxide. The layers were separated and the aqueous portion was extracted with ethyl acetate (500 mL) and ethyl ether (500 mL). The combined organic layer and extracts were dried over sodium sulfate, filtered, and evaporated under vacuum to a white solid (92.3 g). The solid was dissolved in hot ethyl acetate (250 mL), and the solution diluted with hot hexanes (400 mL) and left overnight in a refrigerator. The crystals

of (9E)-9-deoxo-9-hydroxyiminoerythromycin A were collected, washed with ice-cold hexane (250 mL), and dried under vacuum to afford a white solid (88.5 g).

IR ($CH_2Cl_2$) 3560, 3400 (br), 2980, 2950, 1735, 1460, 1389, 1165, 1110, 1085, 1050, and 1010 $cm^{-1}$.

$^1$H NMR ($CDCl_3$) d 5.05 (dd, H-13), 4.90 (d, H-1″), 4.38(d, H-1′), 4.01 (m, H-5″), 3.99 (d, H-3), 3.74 (m, H-8), 3.66 (s, H-11), 3.54 (d, H-5), 3.45 (m, H-5′), 3.28 (s, $OCH_3$), 3.23 (dd, H-2′), 2.96 (t, H-4″), 2.87 (m, H-2), 2.64 (q, H-10), 2.43 (m, H-3′), 2.32 (d, H-2″eq), 2.27 (s, $N(CH_3)_2$), 1.98 (m, H-4), 1.87 (m, H-14a), 1.63 (m, H-4′eq), and 1.46 (s, 6-$CH_3$).

$^1$H NMR ($CD_3OD$) δ 5.19 (dd, H-13), 4.48 (d, H-1′), 4.15 (dq, H-5″), 3.98 (d, H-3), 3.76 (m, H-8), 3.70 (m, H-5′), 3.67 (s, H-11), 3.58 (d, H-5), 3.33 (s, $OCH_3$), 3.23 (dd, H-2′), 3.01 (d, H-4″), 2.92 (m, H-2), 2.72 (m, H-10), 2.70 (m, H-3′), 2.43 (d, H-2″eq), 2.33 (s, $N(CH_3)_2$), 2.01 (m, H-4), 1.88 (m, H-14a), 1.72 (m, H-4′eq), 1.58 (dd, H-2″ax), 1.48 (m, H-14b), 1.45 (s, 6-$CH_3$), 1.26 (d, 5″-$CH_3$), 1.23 (s, 3″-$CH_3$), 1.14 (s, 12-$CH_3$), 1.10 (d, 4-$CH_3$), 1.05 (d, 8-$CH_3$), and 0.84 (t, $CH_2C\underline{H}_3$).

$^{13}$C NMR ($CDCl_3$) δ 175.3, 171.3, 103.1, 96.3, 83.5, 80.3, 78.1, 77.1, 75.1, 74.3, 72.6, 71.2, 70.9, 68.8, 65.4, 65.3, 49.4, 44.6, 40.3, 38.8, 37.8, 35.1, 32.6, 29.2, 27.0, 25.4, 21.5, 21.3, 18.7, 18.6, 16.3, 14.3, 10.6, and 9.3.

$^{13}$C NMR ($CD_3OD$) δ 177.5, 171.6, 104.0, 98.0, 84.2, 81.2, 79.3, 78.3, 76.3, 74.2, 72.9, 72.2, 69.0, 66.7, 65.2, 50.0, 46.3, 40.7, 39.3, 36.2, 32.0, 27.4, 26.7, 22.3, 22.0, 21.6, 19.3, 19.1, 17.3, 16.6, 14.8, 11.2, and 10.2.

EI Mass Spectrum, m/e 748, 590, 574, 462, 431, 416, 398, 174, 159, 158, and 116.

Example 2

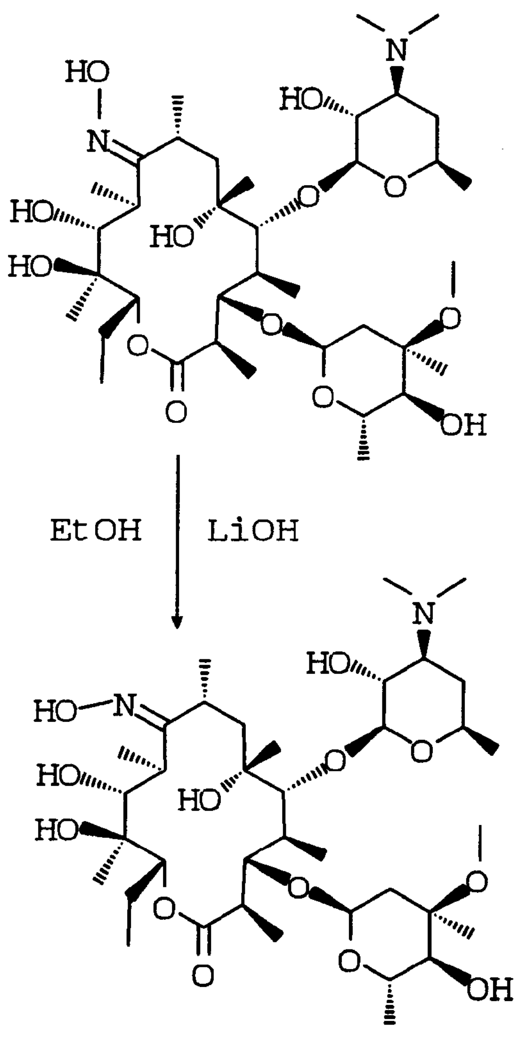

Conversion of 9-Deoxo-9(E)-hydroxyiminoerythromycin A to 9-Deoxo-9(Z)-hydroxyiminoerythromycin A

Method 1:

9-Deoxo-9(E)-hydroximinoerythromycin A (20.0 g, 26.7 mMol) was added to a stirred solution of lithium hydroxide monohydrate (2.25 g, 53.5 mMol) in absolute ethanol (200 mL). The solution was blanketed with nitrogen and stirred overnight at room temperature. The solvents were evaporated under vacuum and the residue was partitioned between ethyl acetate (200 mL) and brine (120 mL). The pH of the mixture was adjusted from 11 to 9.3 with 2 N hydrochloric acid. The ethyl acetate was removed and the brine was re-extracted with more ethyl acetate (2 x 200 mL). The combined ethyl acetate extracts were washed with brine (100 mL), dried with anhydrous magnesium sulfate, filtered and evaporated under vacuum to a foam (ca. 20 g).

The crude oxime mixture was dissolved in methylene chloride (220 mL) and stirred for 1 hour at room temperature to give a filterable, white solid (18.7 g). This material was dissolved in ethyl acetate (100 mL), diluted with nitromethane (100 mL), and 50 mL of solvent was evaporated under vacuum. Additional nitromethane (50 mL) was added and 80 mL of solvent was evaporated under vacuum. The solution was seeded with the (9Z)-isomer and stirred at room temperature for 3 hours. The resulting suspension was filtered and the solids were rinsed with nitromethane (20 mL) and dried under a stream of nitrogen to afford (9Z)-9-deoxo-9-hydroxyiminoerythromycin A (14.8 g, 74% yield) as a white solid.

MP 157-164°C.

IR ($CHCl_3$) 3680, 3435 (br), 2970, 2940, 1725, 1455, 1375, 1345, 1165, 1105, 1085, 1045, 1005, and 950 $cm^{-1}$.

$^1$H NMR ($CDCl_3$) δ 5.01 (dd, H-13), 4.87 (d, H-1″), 4.40 (d, H-1′), 3.98 (m, H-3 and H-5″), 3.80 (s, H-11), 3.49 (m, H-5 and H-5′), 3.27 (s, $OCH_3$), 3.21 (dd, H-2′), 2.99 (m, H-4″), 2.8 (m, H-8, H-2 and H-10), 2.74 (m, H-10), 2.43 (m, H-3′), 2.32 (d, H-2″eq), 2.27 (s, $N(CH_3)_2$), 1.91 (m, H-4), 1.87 (m, H-14eq), 1.63 (m, H-4′eq), 1.51 (m, H-2″ax and H-7), 1.42 (m, H-14ax), 1.37 (s, 6-$CH_3$), 1.28 (d, 10-$CH_3$), 1.24 (d, 5″-$CH_3$), 1.19 (s, 3″-$CH_3$), 1.18 (d, 5′-$CH_3$), 1.12 (d, 2-$CH_3$), 1.11 (s, 12-$CH_3$), 1.08 (d, 8-$CH_3$), 1.04 (d, 4-$CH_3$), and 0.79 (t, $CH_2\underline{CH_3}$).

$^1$H NMR ($CD_3OD$) δ 5.20 (br d, H-13), 4.50 (br d, H-1′), 4.16 (dq, H-5″), 4.02 (d, H-3), 3.70 (m, H-5′), 3.56 (br d, H-5), 3.34 (s, $OCH_3$), 3.25 (dd, H-2′), 3.03 (d, H-4″), 2.87 (m, H-8), 2.84 (m, H-2), 2.73 (m, H-3′), 2.44 (d, H-2″eq), 2.33 (s, $N(CH_3)_2$), 1.97 (m, H-4), 1.88 (m, H-14eq), 1.73 (m, H-4′eq), 1.64 (m, H-7), 1.59 (dd, H-2″ax), 1.47 (m, H-14ax), 1.36 (br s, 6-$CH_3$), 1.28 (d, 5″-$CH_3$), 1.24 (s, 3″-$CH_3$), 1.18 (m, 5′-$CH_3$, 2-$CH_3$, 8-$CH_3$ and 10-$CH_3$), 1.13 (s, 12-$CH_3$), 1.08 (d, 4-$CH_3$), and 0.86 (t, $CH_2\underline{CH_3}$).

$^{13}$C NMR ($CDCl_3$) δ 176.2, 168.2, 102.8, 95.9, 83.6 (br), 79.3 (br), 77.9, 77.3, 75.2, 75.1, 72.7, 71.0, 70.9, 68.8, 65.5, 65.3, 49.4, 40.2, 39.9 (br), 37.8 (br), 35.7 (br), 34.9, 34.1 (br), 28.9, 26.0 (br), 21.4, 21.3, 19.8 (br), 18.4, 16.8, 15.3 (br), 10.7, and 9.2.

$^{13}$C NMR ($CD_3OD$) δ 177.7, 170.0, 103.9, 97.7, 84.3 (br), 80.7, 79.2, 78.1, 77.0 (br), 76.1, 74.1, 72.8, 71.7 (br), 69.2, 66.7, 65.1, 49.9, 46.2 (br), 41.8 (br), 40.8, 40.5 (br), 36.0, 33.8 (br), 31.9, 26.7 (br), 22.8, 21.8, 21.7 (br), 21.6, 19.1, 17.5, 15.8 (br), 12.2 (br), 11.3, and 10.1.

FAB mass spectrum:
m/e 749, 591, 416, 398, 174, 159, 158, and 116.

Elemental Analysis:

| | | | |
|---|---|---|---|
| Calculated for $C_{37}H_{68}N_2O_{13}$: | | | |
| | C, 59.34; | H, 9.15; | N, 3.74. |
| Found: | C, 59.12; | H, 8.80; | N, 3.82. |

Method 2: 1.0 LiOH in EtOH

(9E)-9-Deoxo-9-hydroxyiminoerythromycin A (255 mg, 0.34 mmol) was added to a solution of lithium hydroxide monohydrate (14.3 mg, 0.34 mmol) in absolute ethanol (2.55 mL). The resulting solution was stirred at room temperature for 25 hours, and then stored in a freezer at -20°C for 68 hours. After warming to room temperature, the solution was evaporated under reduced pressure to remove the solvent. The residue was stirred with saturated aqueous sodium chloride (5 mL) and ethyl acetate (5 mL) while the pH was adjusted to 9.2 by addition of dilute hydrochloric acid. After shaking, the phases were separated and the aqueous portion extracted with more ethyl acetate (2 x 2.5 mL). The combined ethyl acetate extracts were washed with saturated sodium chloride solution (4 mL), dried over magnesium sulfate, filtered and evaporated at reduced pressure

to afford a white foam (263 mg). Examination of this material by $^1H$ NMR spectroscopy revealed a 31:69 mixture of (9E)-9-deoxo-(9E)-hydroxyiminoerythromycin A and (9Z)-9-deoxo-9-hydroxyiminoerythromycin A.

Method 3: 2.0 LiOH in EtOH

(9E)-9-Deoxo-9-hydroxyiminoerythromycin A (291 mg, 0.333 mmol) was added to a solution of lithium hydroxide monohydrate (32.6 mg, 0.776 mmol) in absolute ethanol (2.9 mL). The resulting solution was stirred at room temperature under a nitrogen atmosphere for 22.5 hours. The solvent was evaporated at reduced pressure and the residue stirred with ethyl acetate (5 mL) and saturated aqueous sodium chloride (5 mL) while adjusting the pH to 9 by addition of 2N hydrochloric acid. The mixture was shaken, the phases separated, and the aqueous portion extracted with more ethyl acetate (2 x 2.5 mL). The combined ethyl acetate extracts were washed with saturated sodium chloride solution (4 mL), dried with magnesium sulfate, filtered and evaporated under vacuum to a white foam (299 mg). This material was shown by $^1H$ NMR to be a 21:79 mixture of (9E)-9-deoxo-9-hydroxyiminoerythromycin A and (9Z)-9-deoxo-9-hydroxyiminoerythromycin A.

Method 4: 3.0 LiOH in EtOH

(9E)-9-Deoxo-9-hydroxyiminoerythromycin A (239 mg, 0.319 mmol) was was added to a mixture of lithium hydroxide monohydrate (40.2 mg, 0.957 mmol) in absolute ethanol (2.4 mL), and the resulting solution was stirred at room temperature and under a nitrogen atmosphere for 21.7 hours. Workup as described in method 3 afforded a white foam (236 mg) shown by $^1H$ NMR to consist of a 19:81 mixture of (9E)-9-deoxo-9-hydroxyiminoerythromycin A and (9Z)-9-deoxo-9-hydroxyiminoerythromycin A.

Method 5: 2.0 NaOEt in EtOH

Freshly cut sodium metal (48 mg, 2.087 mmol) was dissolved in absoute ethanol (7.8 mL) under a nitrogen atmosphere. (9E)-9-Deoxo-9-hydroxyiminoerythromycin A (782 mg, 1.043 mmol) was added and the resulting solution was stirred at room temperature. A crystalline precipitate, identified as the starting oxime by thin layer chromatography, appeared after a few hours. After stirring overnight, the mixture was once again a clear solution. After 54 hours, approximately half (3.9 mL) of the reaction mixture was removed and evaporated under reduced pressure. The gummy residue was stirred with ethyl acetate (5 mL) and saturated aqueous sodium chloride (5 mL) while the pH was adjusted to 9.2 by addition of dilute hydrochloric acid (2N and 0.2N solutions). The mixture was shaken, the layers separated, and the aqueous portion extracted with more ethyl acetate (2 x 2.5 mL). The combined ethyl acetate solution was washed with saturated brine (5 mL), dried with magnesium sulfate, filtered and evaporated under reduced pressur to a white foam (361 mg). This material was shown by $^1H$ NMR spectroscopy to consist of a 22:78 mixture of the (9E) and (9Z) isomers of 9-deoxo-9-hydroxyiminoerythromycin A.

Method 6: 2.0 NaOH in EtOH

The remaining half of the reaction mixture from method 5 was treated with water (0.0188 mL, 1.04 mmol) to give a solution effectively consisting of sodium hydroxide and oxime in ethanol. The solution was stirred at room temperature for 23 hours, then worked up as described in method 5 to give a white foam (402 mg). This material was shown by $^1H$ NMR to consist of a 24:76 mixture of the (9E) and (9Z) isomers of 9-deoxy-9-hydroxyiminoerythromycin A.

Method 7: 2.0 LiOH in MeOH

A solution of lithium hydroxide monohydrate (37 mg, 0.88 mmol), (9E)-9-deoxo-9-hydroxyiminoerythromycin A (330 mg, 0.44 mmol), and methanol (3.3 mL) was stirred at room temperature for 65.5 hours. The solution was then stored at -20°C for 13 days before warming to room temperature and evaporating the solvent at reduced pressure. The residue was stirred with ethyl acetate (5 mL) and saturated brine (5 mL) while adjusting the pH to 9.2 by addition of dilute hydrochloric acid. The mixture was shaken, the layers separated and the aqueous portion extracted with more ethyl acetate (2 x 2.5 mL). The combined ethyl acetate solution was washed with saturated brine (5 mL), dried with magnesium sulfate, and evaporated under vacuuum to provide a white foam (324 mg). NMR analysis of this material indicated a 45:55 ratio of (9E) to (9Z) 9-deoxo-9-hydroxyiminoerythromycin A products.

Method 8: 2.0 NaOMe in MeOH

A solution of (9E)-9-deoxo-9-hydroxyiminoerythromycin A (375 mg, 0.5 mmol) in anhydrous methanol (3.5 mL) was cooled in an ice bath and stirred under a nitrogen atmosphere while methanolic sodium methoxide (0.23 mL of a 25 wt % solution, 1.01 mmol) was added by syringe. The cooling bath was removed and the solution was stirred at room temperature under a nitrogen atmosphere for 66 hours. The solution was then stored at -20°C for 13.3 days before being processed to a white foam (329 mg) as described in method 7. The product consisted of a 35:65 mixture of (9E)-deoxo-9-hydroxyiminoerythromycin A and (9Z)-9-deoxo-9-hydroxyiminoerythromycin A as determined by $^{1}H$ NMR spectroscopy.

Method 9: 10.0 NaOMe in MeOH

A solution of (9E)-9-deoxo-9-hydroxyiminoerythromycin A (100 mg, 0.134 mmol) in anhydrous methanol (4.70 mL) was treated with sodium methoxide (0.305 mL of a 25 wt. % solution in methanol, 1.335 mmol) and stirred at room temperature for 74.5 hours. The solvent was evaporated under reduced pressure and the residue stirred with ethyl acetate (5 mL) and brine (5 mL) while adjusting the pH of the aqueous layer to 9.4 with 2N hydrochloric acid. The mixture was shaken, the layers separated and the aqueous portion extracted with more ethyl acetate (2 x 2.5 mL). The combined ethyl acetate solution was washed with brine (5 mL), dried with magnesium sulfate, filtered and evaporated at reduced pressure to afford a white foam (102 mg). This material was shown by $^{1}H$ NMR spectroscopy to consist of a 26:74 mixture of the (9E) and (9Z) isomers of 9-deoxo-9-hydroxyiminoerythromycin A.

Method 10: 2.0 LiOH in iPrOH

(9E)-9-Deoxo-9-hydroxyiminoerythromycin A (279 mg, 0.361 mmol) was added to a partial solution of lithium hydroxide monohydrate (30.3 mg, 0.721 mmol) in isopropanol (2.7 mL), and the mixture was stirred at room temperature in a capped flask. A fine white precipitate formed in a few minutes and, after stirring overnight, the mixture was a hazy suspension. After 21 hours, the mixture was transferred to a freezer at -20°C and stored there for 15 days. After warming to room temperature, the solvent was evaporated under vacuum and the residue stirred with ethyl acetate (5 mL) and saturated brine (5 mL) while adjusting the pH to 9.2 with dilute hydrochloric acid. The mixture was shaken, the layers separated, and the aqueous phase extracted with more ethyl acetate (2 x 2.5 ml). The combined ethyl acetate solution was washed with saturated brine (4 mL), dried with magnesium sulfate, filtered and evaporated under vacuum to afford a white foam (249 mg). The product consisted of a 26:74 mixture of (9E)-9-deoxo-9-hydroxyimino-erythromycin A and (9Z)-9-deoxo-9-hydroxyimino-erythromycin A as determined by $^{1}H$ NMR spectroscopy.

Method 11: 1.0 LiOH in MeCN

A mixture of (9E)-9-deoxo-9-hydroxyimino-erythromycin A (500 mg, 0.668 mmol), lithium hydroxide monohydrate (28 mg, 0.668 mmol), and absolute ethanol (5 mL) was stirred at room temperature for 10 minutes to give a solution. The solution was evaporated under reduced pressure to afford a residue that was twice diluted with ethanol (10 mL) and evaporated at reduced pressure and then suspended in anhydrous acetonitrile (5 mL) and evaporated at reduced pressure. The solid residue was suspended in anhydrous acetonitrile (5 mL) and the mixture was stirred at room temperature for 18 days. The solvent was evaporated under reduced pressure and the residue was stirred with ethyl acetate (5 mL) and saturated aqueous sodium chloride solution (5 mL) while adjusting the pH of the aqueous phase to 9.5 by addition of dilute hydrochloric acid. The mixture was shaken, the layers separated, and the aqueous portion was extracted with additional ethyl acetate (2 x 2.5 mL). The combined ethyl acetate solution was washed with brine (5 mL), dried over magnesium sulfate, filtered and evaporated under reduced pressure to afford a foam (442 mg). This material was shown by $^{1}H$ NMR spectroscopy to consist of a 44:56 mixture of the (9E) and (9Z) isomers of 9-deoxo-9-hydroxyiminoerythromycin A.

Method 12: 1.0 LiOH in DMF

A mixture of (9E)-9-deoxo-9-hydroxyiminoerythromycin A (500 mg, 0.668 mmol), lithium hydroxide monohydrate (28 mg), and dimethylformamide (5 mL) was stirred at room temperature in a capped flask. After a few hours, the initial suspension gave way to a solution. After stirring for 18 days and 18 hours, the solution was evaporated under reduced pressure and the residue was processed as described in method 11 to afford a foam (402 mg). Analysis of this material by $^{1}H$ NMR spectroscopy indicated a 62:38 mixture of the (9E) and (9Z) isomers of 9-deoxo-9-hydroxyiminoerythromycin A.

Method 13: 1.2 $LiN(SiMe_3)_2$in MeCN

A suspension of (9E)-9-deoxo-9-hydroxyiminoerythromycin (500 mg, 0.668 mmol) in anhydrous acetonitrile (4 mL) was treated with lithium hexamethyldisilazide (0.80 mL of a 1M solution in hexane, 0.80 mmol). The resulting suspension rapidly gave way to a solution which reformed a suspension after stirring several days at room temperature. After 18 days and 19 hours, the reaction mixture was worked up as described in method 11 to afford a foam (423 mg). This material was shown by $^1H$ NMR spectroscopy to be a 50:50 mixture of (9E)-9-deoxo-9-hydroxyiminoerythromycin A and (9Z)-9-deoxo-9-hydroxyimino-erythromycin A.

Example 3

Crystallization of 9-Deoxo-9(Z)-hydroxyimino-erythromycin A

A 3:1 mixture (30.0 g) of (9Z)-9-deoxo-9-hydroxyiminoerythromycin A and 9-deoxo-9(E)-hydroxyiminoerythromycin A was added over 2 minutes to well stirred ethyl acetate (60ml). After obtaining a solution, methylene chloride (120 mL) was rapidly added and the resulting suspension was stirred in an ice bath for one hour. The precipitate was filtered off, washed with methylene chloride (60 mL), and dried under a stream of nitrogen to afford an 86:14 mixture (26.5 g) of of 9-deoxo-9(Z)-hydroxyiminoerythromycin A and 9-deoxo-9(E)-hydroxyiminoerythromycin A.

A solution of the above solid in ethyl acetate (60 mL) was diluted with methylene chloride (120mL). The resulting suspension was cooled in an ice bath for one hour and then filtered. The collected solid was rinsed with methylene chloride (60 mL) and dried under a stream of nitrogen to afford a 95:5 mixture (23.4 g) of (9Z)-9-deoxo-9-hydroxyiminoerythromycin A and (9E)-9-deoxo-9-hydroxyiminoerythromycin A.

Example 4

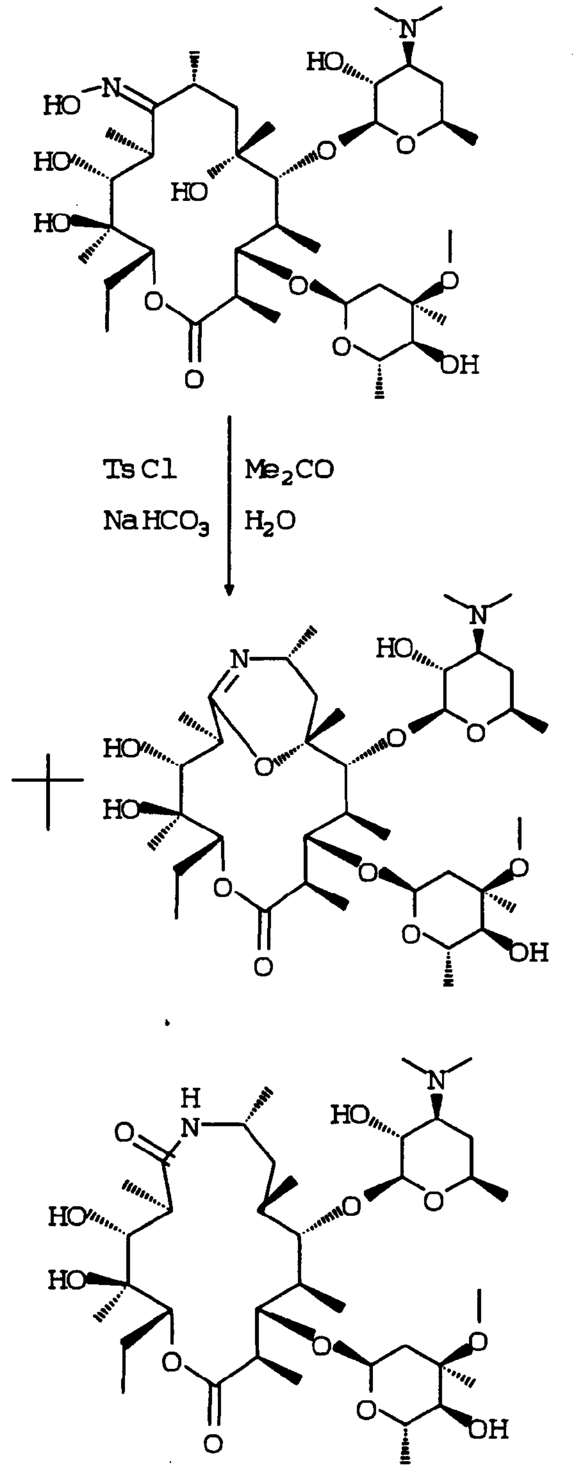


Synthesis of 8a-Aza-8a-homoerythromycin A and 9-Deoxo-6,9-epoxy-8a,9-didehydro-8a-aza-8a-homoerythromycin A by the Beckmann Rearrangement of 9-Deoxo-9(Z)-hydroxyiminoerythromycin A

Method 1:

(9Z)-9-Deoxo-9-hydroxyiminoerythromycin A (200 mg, 0.27 mMol) was dissolved in acetone (2 mL) and

the resulting solution was cooled in an ice-bath and stirred under a nitrogen atmosphere. A solution of sodium bicarbonate (84 mg, 1.0 mMol) in water (2 mL) was added followed by the dropwise addition of an acetone solution (2 mL) of p-toluenesulfonyl chloride (100 mg, 0.53 mMol) over 5 minutes.

After stirring for 1.5 hours at 0-5°C, the mixture was diluted with dichloromethane (10 mL) and water (5 mL), and the pH was adjusted from 10 to 5.5 with 2N HCl. The dichloromethane layer was discarded and the aqueous layer was washed with additional dichloromethane (2 x 10 mL) which was also discarded. Dichloromethane (10 mL) was added to the aqueous layer and the pH was adjusted to 8.5 with 2.5 N NaOH. The dichloromethane layer was removed and the aqueous layer was extracted with more dichloromethane (2 x 20 mL). The combined dichloromethane extracts were dried over anhydrous magnesium sulfate, filtered and evaporated under vacuum to give a mixture of the title compounds as a foam (150 mg).

The above mixture was purified by preparative layer chromatography (two 0.1 mm x 20 x 20 cm Analtech silica gel GF plates, developing and eluting with 60:10:1 dichloromethane-methanol-concentrated ammonium hydroxide) to afford 8a-aza-8a-homoerythromycin A (95 mg, 48% yield) and 9-deoxo-6-deoxy-6,9-epoxy-8a,9-didehydro-8a-aza-8a-homoerythromycin (33 mg, 17% yield).

Method 2:

A solution of p-toluenesulfonyl chloride (1.00 g, 5.2 mmol) in acetone (20 mL) was added to a solution of sodium bicarbonate (0.90 g, 10.7 mmol) in water (20 mL). The resulting suspension was cooled in a -10°C bath and stirred while a solution of (9Z)-9-deoxo-9-hydroxyiminoerythromycin A (2.00 g, 2.7 mmol) in acetone (20 mL) was added slowly over 75 minutes. The mixture was stirred at -10°C for 5 hours, then warmed to 0°C over 10 minutes and stirred at 0-5°C for 30 minutes. The mixture was evaporated under vacuum to remove the acetone. The aqueous residue was diluted with water (40 mL) and dichloromethane (60 mL) and stirred while the pH was adjusted to 5.5 with dilute hydrochloric acid. The aqueous layer was separated, washed with dichloromethane (60 mL), layered with dichloromethane (60 mL), and stirred while the pH was brought to 9 with dilute aqueous sodium hydroxide. The layers were separated and the aqueous portion extracted with more dichloromethane (2 x 50 mL). The combined pH 9 extracts were dried over magnesium sulfate, filtered and evaporated under reduced pressure to afford a gum (1.97 g) which was shown by $^1H$ NMR spectroxcopy to be a 1:1 mixture of 8a-aza-8a-homoerythromycin A and 9-deoxo-6-deoxy-6, 9-epoxy-8a,9-didehydro-8a-aza-8a-homoerythromycin A.

The crude product mixture was dissolved in 120:10:1 dichloromethanemethanol-conc. aqueous ammonium hydroxide (5 mL) and loaded onto a column of silica gel (4 x 16 cm). The column was eluted with 120:10:1 dichloromethane-methanol-ammonium hydroxide. After a 150 mL forerun, 15 mL fractions were collected. Fractions 9-13 were combined and evaporated under vacuum to afford 9-deoxo-6-deoxy-6, 9-epoxy-8a,9-didehydro-8a-aza-ua-homoerythromycin A (ca. 500 mg) and fractions 22-26 were combined and evaporated to afford 8a-aza-8a-homoerythromycin A (ca. 500 mg). The later product was crystallized from ether to give the amide (ca. 130 mg) as a white solid.

Physical data for 9-deoxo-6-deoxy-6,9-epoxy-8a, 9-didehydro-8a-aza-8a-homoerythromycin A:

IR ($CHCl_3$) 3550, 3440 (br), 2970, 2940, 2880, 1725, 1665, 1455, 1375, 1345, 1325, 1240, 1170, 1105, 1080, 1050, 1015, 995, and 955 $cm^{-1}$.

$^1H$ NMR ($CDClC_3$) δ 5.02 (d, H-1″), 4.90 (dd, H-13), 4.48 (d, H-1′), 4.09 (dq, H-5″), 4.02 (t, H-3), 3.81 (d, H-5), 3.53 (m, H-5′), 3.49 (d, H-11), 3.43 (m, H-8), 3.35 (s, $OCH_3$), 3.20 (dd, H-2′), 3.07 (t, H-4″), 2.75 (dq, H-2), 2.68 (dq, H-10), 2.52 (ddd, H-3′), 2.43 (d, H-2″eq), 2.28 (s, $N(CH_3)_2$), 1.98 (ddq, H-4), 1.91 (m, H-14a), 1.90 (dd, H-7a), 1.68 (ddd, H-4′eq), 1.62 (dd, H-2″ax), 1.46 (m, H-14b), 1.39 (s, 6-$CH_3$), 1.32 (d, 5″-$CH_3$), 1.27 (s, 3″-$CH_3$), 1.24 (m, H-7b), 1.22 (d, 5′-$CH_3$), 1.21 (m, H-4′ax), 1.16 (d, 10-$CH_3$), 1.15 (d, 8-$CH_3$), 1.15 (s, 12-$CH_3$), 1.14 (d, 2-$CH^3$), 1.08 (d, 4-$CH_3$), and 0.87 (t, $CH_2\underline{CH_3}$).

$^{13}C$ NMR ($CDCl_3$) δ 177.6, 160.6, 102.4, 94.6, 80.1, 78.9, 77.9, 77.4, 76.5, 75.7, 73.0, 70.6, 70.0, 68.8, 65.8, 65.6, 49.4, 44.9, 44.0, 42.3, 42.1, 40.3, 34.5, 32.0, 28.5, 23.8, 22.4, 21.5, 21.3, 21.0, 18.2, 17.0, 16.4, 12.5, 10.8, and 8.4.

FAB mass spectrum, m/e 731, 713, 602, 573, 555, 398, 159, 158, and 116.

Physical data for 8a-aza-8a-homoerythromycin A:

MP 170-176°C.

IR ($CHCl_3$) 3500 (br), 3430, 3320, 2970, 2935, 2880, 1730, 1630, 1560, 1525, 1455, 1375, 1325, 1280, 1170, 1160, 1105, 1085, 1045, 1010 and 995 $cm^{-1}$.

$^1H$ NMR ($CDCl_3$) δ 5.89 (br d, NH), 5.07 (d, H-1″), 4.92 (dd, H-13), 4.43 (d, H-1′), 4.35 (d, H-3), 4.21 (m,

H-8), 4.01 (dq, H-5″), 3.58 (d, H-5), 3.50 (m, H-5′), 3.50 (s, H-11), 3.32 (s, $OCH_3$), 3.21 (dd, H-2′), 3.03 (t, H-4″), 2.62 (dq, H-2), 2.54 (m, H-3′), 2.35 (m, H-10), 2.35 (s, $N(CH_3)_2$), 2.31 (d, H-2″eq), 1.90 (m, H-4), 1.89 (m, H-14a), 1.75 (br d, H-4′eq), 1.57 (dd, H-2″ax), 1.51 (m, H-7a and H-7b), 1.44 (m, H-14b), 1.43 (s, 6-$CH_3$), 1.30 (d, 5″-$CH_3$), 1.24 (s, 3″-$CH_3$), 1.23 (m, H-4′ax), 1.23 (d, 5′-$CH_3$), 1.20 (d, 8-$CH_3$), 1.19 (d, 10-$CH_3$), 1.18 (d, 2-$CH_3$), 1.09 (s, 12-$CH_3$), 1.05 (d, 4-$CH_3$), and 0.89 (t, $CH_2C\underline{H}_3$).

$^{13}C$ NMR ($CDCl_3$) $\delta$ 177.6, 176.6, 102.7, 94.2, 83.0, 77.9, 77.0, 76.6, 74.6, 73.7, 72.9, 70.0, 69.8, 68.8, 65.8, 65.2, 49.2, 45.8, 43.2, 42.4, 41.0, 40.4, 40.1, 34.5, 28.3, 27.6, 23.1, 21.7, 21.5, 21.2, 18.0, 16.1, 14.6, 11.2, 10.0, and 9.1.

FAB Mass spectrum, m/e 749, 731, 591, 589, 573, 416, 174, 159, 158 and 117.

Elemental Analysis. Calculated for $C_{37}H_{68}N_2O_{13}$: C, 59.34; H, 9.15; N, 3.74. Found: C, 59.24; H, 9.15; N, 3.44. Loss on drying at 120°C, 3.11%.

EXAMPLE 5

Synthesis of 9-Deoxo-6-deoxy-6,7-epoxy-8a,9-didehydro-8a-aza-8a-homoerythromycin A and 9-Deoxo-izdeoxy-9,12-epoxy-8a,9-didehydro-8a-aza-8a-homoerythromycin A by Beckmann Rearrangement of (9Z)-9-Deoxo-9-hydroximino-erythromycin A

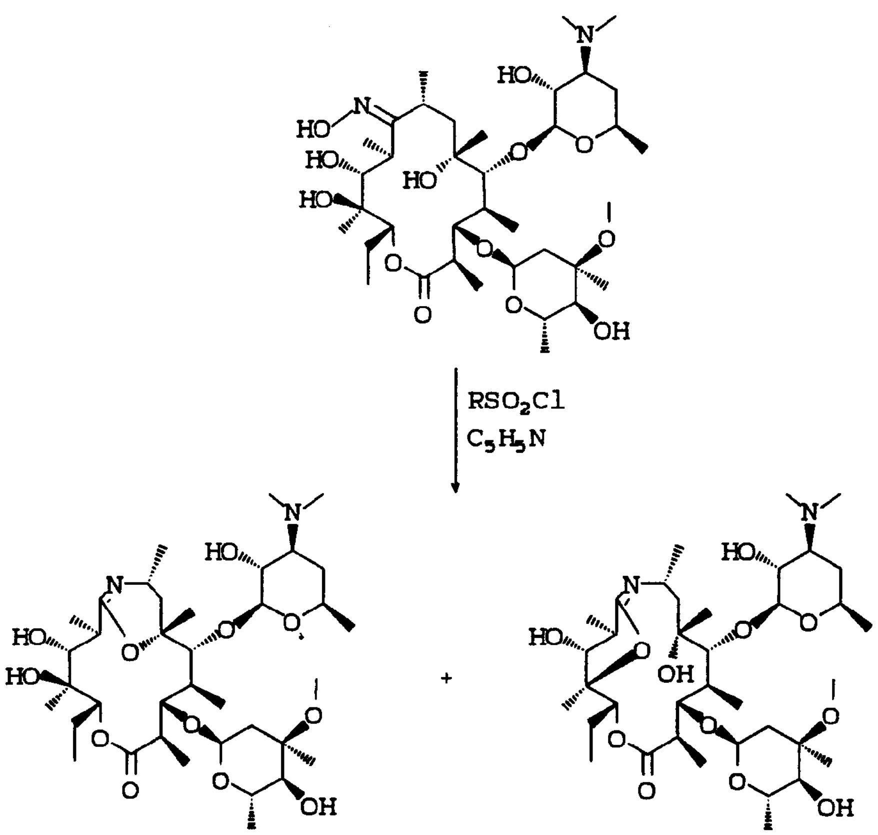


Method 1

A solution of p-toluenesulfonyl chloride (15.0 g, 0.079 mol) in diethyl ether (50 mL) was added dropwise over 8 minutes to an ice-cold, stirring solution of (9Z)-9-deoxo-9-hydroxyiminoerythromycin A (23.2 g, 0.031 mol) in pyridine (180 mL). The resulting solution was stirred at 0-5°C for 2.5 hours, then diluted with dichloromethane (400 mL) and water (500 mL) and basified to pH 9.5 by addition of 5N sodium hydroxide. The layers were separated and the aqueous portion extracted with more dichloromethane (200 mL, 100 mL). The combined dichloromethane extracts were dried over magnesium sulfate, filtered, and evaporated under vacuum to afford an oil. Residual pyridine was removed by twice taking the product up in toluene (100 mL) and evaporating the solvent under vacuum. The resulting foam (21.4 g) was shown by $^1H$ NMR spectroscopy to be a 26:74 mixture of 9-deoxo-6-deoxy-6,9-epoxy-8a, 9-didehydro-8a-aza-8a-homoerythromycin A and 9-deoxo-12-deoxy-9,12-epoxy-8a,9-didehydro-8a-aza-8a-homoerythromycin A.

Method 2

A solution of p-toluenesulfonyl chloride (160 mg, 0.84 mmol) in diethyl ether (0.5 mL) was added rapidly to an ice-cold solution of (9Z)-9-deoxo-9-hydroxyiminoerythromycin A (250 mg, 0.33 mmol) in pyridine (2.0 mL). The resulting solution was stirred at 0-5°C for 1.5 hours, then diluted with dichloromethane (4 mL) and water (4 mL) and basified to pH 9.5 by addition of 5N sodium hydroxide. The layers were separated and the aqueous portion extracted with more dichloromethane (2 x 4 mL). The combined dichloromethane extracts were dried over magnesium sulfate, filtered, evaporated under vacuum and stripped with hexane (4 x 15 mL) to afford a yellow solid (260 mg). This material was shown by 1H NMR spectroscopy to be a 25:75 mixture of 9-deoxo-6-deoxy-6,9-epoxy-6,9-epoxy-8a,9-anhydro-8a-homoerythromycin A and 9-deoxo-12-deoxy-9,12-epoxy-8a, 9-didehydro-8a-aza-8a-homoerythromycin A.

Method 3

A solution of p-toluenesulfonyl chloride (160 mg, 0.84 mmol) in acetonitrile (0.5 mL) was added rapidly to an ice-cold solution of (9Z)-9-deoxo-9-hydroxyiminoerythromycin A (250 mg, 0.33 mmol) in pyridine (2.0 mL). The resulting solution was stirred at 0-5°C for 80 minutes, then diluted with dichloromethane (4 mL) and water (5 mL) and basified to pH 9.5 by addition of 5N sodium hydroxide. The layers were separated and the aqueous portion extracted with more dichloromethane (2 x 4 mL). The combined dichloromethane extracts were dried over magnesium sulfate, filtered, and evaporated under vacuum to a foam which was stripped with toluene (2 x 10 mL) and hexanes (10 mL) to afford a solid (230 mg). This material was shown by $^{1}H$ NMR spectroscopy to be a 33:67 mixture of 9-deoxo-6-deoxy-6,9-epoxy- and 9-deoxo-12-deoxy-9,12-epoxy-8a,9-dide-hydro-8a-aza-8a-homoerythromycin A.

Method 4

A solution of p-toluenesulfonyl chloride (160 mg, 0.84 mmol) in toluene (0.5 mL) was added rapidly to an ice-cold solution of (9Z)-9-deoxo-9-hydroxyiminoerythromycin A (250 mg, 0.33 mmol) in pyridine (2.0 mL). The resulting solution was stirred at 0-5°C for 90 minutes, then diluted with dichloromethane (4 mL) and water (4 mL) and basified to pH 9.5 by addition of 1N sodium hydroxide. The layers were separated and the aqueous portion extracted with more dichloromethane (3 x 4 mL). The combined dichloromethane extracts were dried over magnesium sulfate, filtered, and evaporated under vacuum to a solid (250 mg). This material was shown by $^{1}H$ NMR spectroscopy to be a 27:73 mixture of 9-deoxo-6-deoxy-6,9-epoxy- and 9-deoxo-12-deoxy-9, 12-epoxy-8a,9-dide-hydro-8a-aza-8a-homoerythromycin A.

Method 5

Benezenesulfonyl chloride (0.107 mL, 0.84 mmol) was added by syringe to an ice-cold solution of (9Z)-9-deoxo-9-hydroxyiminoerythromycin A (250 mg, 0.33 mmol) in pyridine (2.0 mL). The resulting solution was stirred at 0-5°C for 75 minutes, then processed as described above to afford a yellow solid (240 mg). This material was shown by $^{1}H$ NMR spectroscopy to be a 31:69 mixture of 9-deoxo-6-deoxy -6,9-epoxy- and 9-deoxo-12-deoxy-9,12-epoxy-8a, 9-didehydro-8a-aza-8a-homoerythromycin A.

Method 6

Methanesulfonyl chloride (0.065 mL, 0.84 mmol) was added by syringe to an ice-cold solution of (9Z)-9-deoxo-9-hydroxyiminoerythromycin A (250 mg, 0.33 mmol) in pyridine (2.0 mL). The resulting solution was stirred at 0-5°C for 2 hours, then processed as described above to afford an off-white solid (246 mg). This material was shown by $^{1}H$ NMR spectroscopy to be a 25:70:5 mixture of 9-deoxo-6-deoxy-6,9-epoxy-8a,9-didehydro-8a-aza-8a-homoerythromycin A, 9-deoxo-12-deoxy-9,12-epoxy-8a, 9-didehydro-8a-aza-8a-homoerythromycin A, and 9-deoxy-12-deoxy-9,12-epoxy-4″-O-methanesulfonyl-8a,9-didehydro-8a-aza-8a-homoerythromycin A.

Method 7

A solution of (9Z)-9-deoxo-9-hydroxyiminoerythromycin A (250 mg, 0.33 mmol) in pyridine (2.0 mL) was cooled in a -20°C bath and treated with methanesulfonyl chloride (0.071 mL, 0.92 mmol). The resulting hazy solution was stirred at -10 to -20°C for 90 minutes, then processed as described above to afford a yellow solid (254 mg). This material was shown by $^1$H NMR spectroscopy to be a 88:12 mixture of 9-deoxo-6-deoxy-6,9-epoxy-8a,9-didehydro-8a-aza-8a-homoerythromycin A and 9-deoxo-12-deoxy-9,12-epoxy-8a, 9-didehydro-8a-aza-8a-homoerythromycin A.

Method 8

A mixture of (9Z)-9-deoxo-9-hydroxyiminoerythromycin A (0.50 g, 0.67 mmol), p-toluenesulfonyl chloride (318 mg, 1.67 mmol) and pyridine (0.162 mL, 2.0 mmol) in dichloromethane (5.0 mL) was stirred at room temperature for 1.5 hours. The mixture was diluted with water and stirred rapidly while adjusting the pH to 11 with 5N sodium hydroxide. The organic phase was separated, dried with magnesium sulfate, filtered and evaporated under reduced pressure to afford a yellow solid (570 mg). Analysis of the crude product by $^1$H NMR spectroscopy revealed a 80:20 mixture of 9-deoxo-6-deoxy-6,9-epoxy-8a, 9-didehydro-8a-aza-8a-homoerythromycin A and 9-deoxo-12-deoxy-9,12-epoxy-8a,9-didehydro-8a-aza-8a-homoerythromycin A.

Example 6

The antibacterial activities of compound (III) against a panel of aerobic Gram positive and negative bacteria is shown in the following Table. The assay employs a liquid turbidimetric microtiter method for the determination of the minimum inhibitory concentration (MIC) in brain heart infusion broth. The MIC endpoint in mcg/ml is defined as the lowest concentration of test compound that completely inhibits the growth (absence of turbidity) of bacteria. The MIC is generally not an absolute value but rather a concentration ranged that falls within a two-fold dilution limit. Generally twelve two-fold dilutions of the test compound are employed with the initial concentration set at 128 mcg/ml.

TABLE 1

In vitro Activity of Compound (III)

MIC Values (mcg/m)

| Microorganism | | (III) |
|---|---|---|
| Enterococcus faecalis | MB 5407 | 16 |
| Enterococcus faecium | MB 5416 | ≤0.06 |
| Streptococcus agalactiae | CL 1343 | 0.25 |
| Staphylococcus aureus | MB 2865 | 1 |
| Staphylococcus epidermidis | MB 5414 | 2 |
| Staphylococcus haemolyticus | MB 5412 | 2 |
| Streptococcus pneumoniae | CL 2883 | ≤0.06 |
| Streptococcus pyogenes | MB 2874 | ≤0.06 |
| Streptococcus pyogenes | MB 5406 | 128 |
| Steptococcus viridans | CL 2943 | 4 |
| Escherichia coli | MB 2884 | 32 |
| Escherichia coli | MB 4926 | 4 |
| Klebsiella pneumoniae | MB 4005 | 64 |
| Yersinia enterocoltica | CL 1598 | 64 |
| Pseudomonas stutzeri | MB 1231 | 0.12 |

(III) 8a-aza-8a-homoerythromycin A.

The compounds of formula (III) are useful as an antibacterial agents both in vitro and in vivo, and their spectrum of activity is similar to that of erythromycin A. Consequently, they can be used for the same purposes, and, in the same manner, as erythromycin A. In general, the antibacterial compounds of formula (III) and salts thereof, exhibit in vitro activity against a variety of Gram-positive microorganisms, e.g. Staphylococcus aureaus and Streptococcus pyogenes, and against certain Gram-negative microorganisms such as those of spherical or ellipsoidal shape (cocci). Their activity is readily demonstrated by in vitro tests against various micro-organisms. Their in vitro activity renders them useful for topical application; for sterilization purposes, e.g., sick-room utensils; and as industrial antimicrobials, for example, in water treatment, slime control, and preservation of paint and wood. The extrapolation of such in vitro tests to support for such utilities for macrolide compounds is taught in U.S. Patent No. 4,518,590. For in vitro use for topical application, it will usually be convenient to prepare pharmaceutical compositions, in which a compound is combined with a pharmaceutically-acceptable carrier or diluent, for example, in the form of ointments and creams. Appropriate carriers and diluents for these purposes include mineral oils and vegetable oils, and solvents such as water, alcohols, and glycols, and mixtures thereof. Such a pharmaceutical composition will normally contain the pharmaceutically-acceptable carrier and a compound of formula II in a weight ratio in the range from 1:4 to 1:200.

Additionally, the antibacterial compounds of formula (III), and the pharmaceutically-acceptable salts thereof are active in vivo, versus a variety of Gram-positive microorganisms, e.g. Staphylococcus aureaus and Streptococcus pyogenes, and also certain Gram-negative microorganisms, via the oral and parenteral routes of administration in animals, including man. Their in vivo activity is more limited than their in vitro activity as regards

susceptible organisms, and it is determined by the usual procedure which comprises infecting mice of substantially uniform weight with the test organism and subsequently treating them orally or subcutaneously with the test compound. Extrapolation of such in vivo tests to support for human utility for macrolide compounds is likewise taught in U.S. Patent No. 4,518,590, cited above.

While the invention has been described, exemplified and illustrated with reference to certain preferred embodiments thereof, those skilled in the art will appreciate that various changes, modifications and substitutions can be made therein without departing from the spirit and scope of the invention.

It is intended, therefore, that the invention be limited only by the scope of the claims which follow and that such claims be interpreted as broadly as is reasonable.

## Claims

1. A method of making a compound of the formula

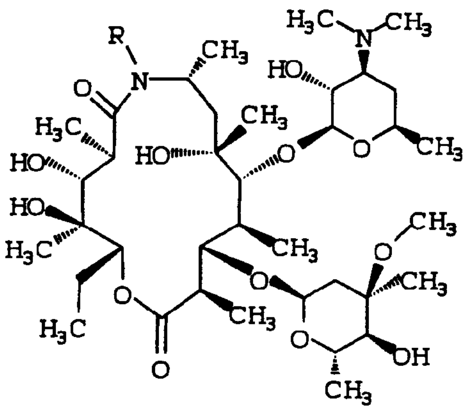

and the pharmaceutically acceptable salts thereof, wherein R is hydrogen or C1-10 alkyl, comprising the step of reacting a compound of the formula

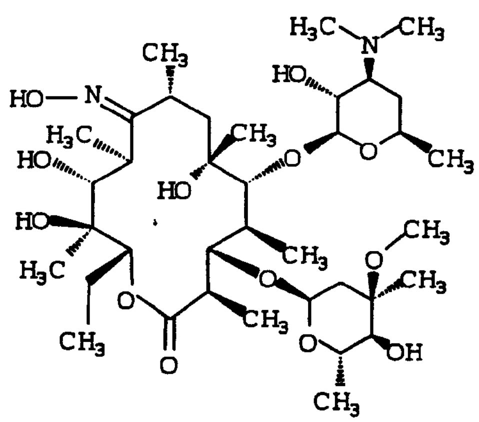

with a suitable acid.

2. A method of making a compound of the formula

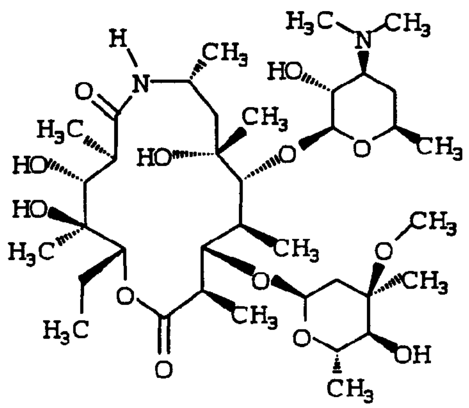

and the pharmaceutically acceptable salts thereof, comprising the step of reacting a compound of the formula

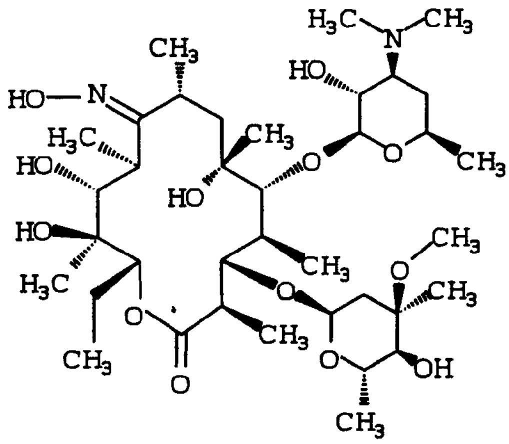

with a suitable acid.

3. The method as claimed in Claim 1 or Claim 2, in which said acid is an arylsulfonyl halide.

4. The method as claimed in Claim 3, in which said acid is p-toluene sulfonylchloride.

5. The method as claimed in Claim 1 or Claim 2, which is additionally carried out in the presence of aqueous sodium bicarbonate and acetone.

6. A method of using a starting compound of the formula

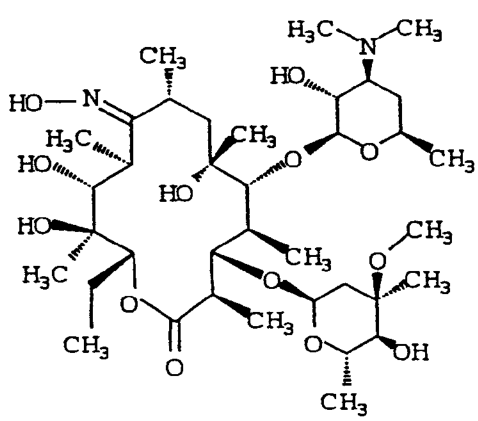

in the synthesis of a compound of the formula

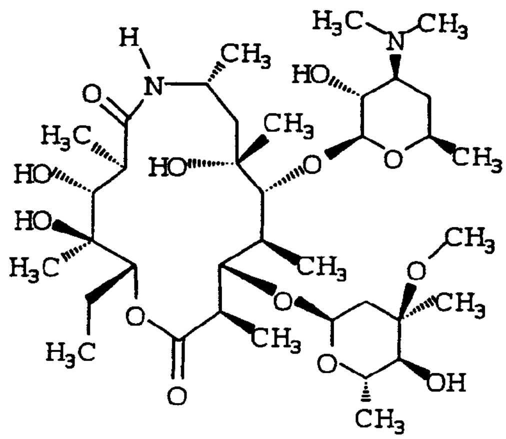

comprising the step of reacting the starting compound with a suitable acid.

7. The method as claimed in Claim 6, in which said acid is an arylsulfonyl halide.

8. The method as claimed in Claim 7, in which said acid is p-toluene sulfonylchloride.

9. The method as claimed in Claim 6, which is further carried out in the presence of acetone and aqueous sodium bicarbonate.

European Patent Office

# EUROPEAN SEARCH REPORT

Application Number

EP 92 30 4524

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| A | JOURNAL OF ANTIBIOTICS.<br>vol. 44, no. 3, March 1991, TOKYO JP<br>pages 313 - 330;<br>J-C. GASC ET AL.: 'New Ether Derivatives of Erythromycin A'<br>*page 314, compound III*<br>* page 315, line 3 - line 6 * | 1 | C07H17/08 |
| A | JOURNAL OF THE CHEMICAL SOCIETY, PERKIN TRANSACTIONS 1.<br>no. 11, 1986, LETCHWORTH GB<br>pages 1881 - 1890;<br>S.DJOKIC ET AL.: 'Erythromycin Series. Part 11. Ring Expansion of Erythromycin A Oxime by the Beckmann Rearrangement'<br>*page 1882, scheme 1, compound (2) ---> compounds (9) and (10)* | 1 | |
| A | EP-A-0 109 253 (PFIZER INC)<br>* page 6, line 20 - line 29 * | 1-9 | |
| | | | TECHNICAL FIELDS SEARCHED (Int. Cl.5 ) |
| A | EP-A-0 410 433 (PLIVA FARMACEUTSKA KEMIJSKA PREHRAMBENA I KOZMETICKA INDUSTRIJA S.P.O.)<br>* page 9, line 36 - line 45 *<br>* abstract * | 1-9 | C07H |
| A | CHEMICAL ABSTRACTS, vol. 98, no. 3, January 1983, Columbus, Ohio, US;<br>abstract no. 17003Z,<br>PLIVA: '11-Aza-10-Deoxo-10-Dihydroerythromycin A'<br>page 542 ;column 1 ;<br>* abstract *<br>& JP-A-8 288 193 (PLIVA FARMACEUTSKIH I KEMIJSKIH PROIZVODA...) | 1 | |

The present search report has been drawn up for all claims

| Place of search | Date of completion of the search | Examiner |
|---|---|---|
| THE HAGUE | 24 JULY 1992 | SCOTT J.R. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)